# EUROPEAN PATENT APPLICATION

(11) **EP 4 292 613 A1**
(43) Date of publication of application: **20.12.2023**
(21) Application number: 22752738.9
(22) Date of filing: 08.02.2022
(51) Int. Cl.: A61K 47/04, A61K 9/70, A61K 31/381, A61K 31/4468, A61K 45/00, A61K 47/06, A61K 47/10, A61K 47/12, A61K 47/14, A61K 47/18, A61K 47/32, A61K 47/34, A61P 25/04, A61P 25/16, A61P 29/00

(54) **ANTIOXIDANT-CONTAINING TRANSDERMAL PREPARATION**

(30) Priority: 10.02.2021 JP 2021020253
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); KUMAMOTO, Satoshi, Kyoto-shi, Kyoto 601-8014 (JP); JIANG, Rongrong, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2022/004911
(87) International publication number: WO 2022/172915

(57) **Abstract**

Provided is a transdermal preparation in which a medicine in an amount sufficient to exhibit a desired medicinal effect is stably retained in a plaster and irritation to the skin is reduced as much as possible. A transdermal preparation including a base; a medicine; and a stabilizer, in which the medicine and the stabilizer are contained in the base, and the stabilizer is selected from the group consisting of thiosulfates, sulfites, bisulfites, and nitrites. The transdermal preparation preferably further includes glycerin.

## Description

### TECHNICAL FIELD

The present invention relates to a transdermal preparation, such as a tape preparation or a patch preparation (that refers to a preparation having a dosage form of a laminate of a medicine-containing reservoir layer and an adhering layer), to be applied to a skin and used for administering a necessary medicine to a subcutaneous tissue or an internal circulatory system via a biological membrane.

### BACKGROUND ART

In the case of oral administration of a medicine, the absorption of the medicine generally depends on the state of the stomach or the intestine, such as the pH value or the presence or absence of contents, and therefore it is difficult to keep the amount of the absorbed medicine constant, and it is also difficult to administer a certain amount of the medicine over a long period of time gradually. In oral administration, the inconstant amount of the absorbed medicine may sometimes cause a rapid increase in the blood concentration, resulting in a side effect, and in the case of a medicine having a short half-life period, the duration of the effective blood concentration may be so short that long retention of a sufficient medicinal effect cannot be achieved. Injection administration further has problems such as pain at the time of administration and inconvenience of the administration method. In the case of a preparation intended for local action, the medicine is to be maintained at a constant concentration in the subcutaneous tissue via the stratum corneum.

Therefore, in order to solve these problems, development of transdermal preparations has been actively promoted because a constant effective blood concentration can be maintained over a long period of time, and improvement in convenience, performance, and the like can be expected. This kind of transdermal preparation is intended to administer a medicine to the internal circulatory system via the stratum corneum of the skin, which originally has a barrier function to prevent foreign substances from entering the body, and therefore it is not always easy to administer the medicine in an amount sufficient to exhibit the intended medicinal effect, and measures are usually taken such as adding an absorption enhancer to the base to increase the penetration of the medicine or increasing the adhering area.

In a case where the transdermal preparation is a patch, the patch has a side effect of stimulating the skin when adhered, and therefore the adhering area is preferably as small as possible. In order to reduce such a side effect, it is desirable that the skin penetration of the medicine be improved by adding an absorption enhancer to reduce the adhering area of the patch. Therefore, many proposals have been made regarding an absorption enhancer for promotion of transdermal absorption of a medicine by adding a specific compound to a preparation (for example, Patent Documents 1 and 2). In a case where the medicine itself is a substance having poor stability, many studies have been made on a stabilizer for prevention of degradation during storage (for example, Patent Document 3).

In the case of a rotigotine-containing transdermal preparation, a method, for producing a transdermal preparation, including a step of mixing rotigotine and an antioxidant at a weight ratio of 1 : 0.0001 to 0.1 is known (Patent Document 4) . The antioxidant in Patent Document 4 is one or more substances selected from the group consisting of tocopherol, esters of tocopherol, ascorbic acid, ascorbyl palmitate, 2,5-dihydroxybenzoic acid, butylhydroxytoluene, butylated hydroxyanisole, and propyl gallate. Furthermore, a rotigotine-containing transdermal patch is known that includes a support, a medicine-containing layer, and a release liner, and the medicine-containing layer includes (1) a rubber-based adhesive, (2) rotigotine or a salt of rotigotine, and (3) a production inhibitor of a degradation product of rotigotine (Patent Document 5). As the production inhibitor of a degradation product of rotigotine in Patent Document 5, mercaptobenzimidazole and sulfites are used.

In the case of a fentanyl-containing transdermal preparation, a patch is known that includes a support and an adhesive layer layered on at least one surface of the support, and the adhesive layer includes at least one selected from the group consisting of fentanyl and salts of fentanyl, an adhesive base, and an antioxidant having a sulfur atom in its molecule (Patent Document 6). In Patent Document 6, the antioxidant having a sulfur atom in its molecule is at least one selected from the group consisting of 2-mercaptobenzimidazole and sodium pyrosulfite.

### PRIOR ART DOCUMENT

### PATENT DOCUMENTS

Patent Document 1: JP H10-507199 A (Japanese Patent No. 3228341)
Patent Document 2: WO 2011/049038 A (Japanese Patent No. 5913981)
Patent Document 3: JP H3-261722 A (Japanese Patent No. 3002493)
Patent Document 4: JP 2017-515871 A
Patent Document 5: JP 2013-079220 A (Japanese Patent No. 5856424)
Patent Document 6: WO 2017/073516 A (Japanese Patent No. 6453481)

### SUMMARY OF THE INVENTION

### PROBLEMS TO BE SOLVED BY THE INVENTION

The present invention relates to a transdermal preparation including a stabilizer. The stabilizer acts to improve the stability of the medicine in the adhesive layer. However, adding the stabilizer to the adhesive base should not cause a decrease in the adhesiveness or an increase in the skin irritation. Therefore, an optimum one is to be selected as the stabilizer in consideration of the target medicine, the adhesive base to be compounded, and the like.

Furthermore, the stabilizer acts on the skin during the time when the transdermal preparation is adhered to the skin, and therefore should not have skin irritation. Many stabilizers have been used to date. Typical stabilizers include antioxidants such as dibutylhydroxytoluene (BHT), tocopherol, vitamin C and its derivatives, and hydroquinone. Thiosulfates and sulfites have strong reducibility and a strong antioxidant effect. However, these compounds are inorganic salts and have poor compatibility with rubber-based adhesives and acryl-based adhesives that are general bases for transdermal preparations, and thus have a drawback that they agglomerate in a particulate form in the base and less likely to exhibit a stabilization effect. In view of the above circumstances, an object of the present invention is to provide a transdermal preparation in which a medicine in an amount sufficient to exhibit a desired medicinal effect is stably retained in a plaster.

### MEANS FOR SOLVING THE PROBLEM

During intensive studies of a novel stabilizer, the present inventors have found that inorganic reducing substances such as thiosulfates and sulfites have excellent stability of many medicines and low skin irritation. These compounds are less likely to be dissolved in many organic solvents, and have extremely poor compatibility with rubber-based adhesives, acryl-based adhesives, and silicone-based adhesives that are bases of a transdermal preparation, and therefore even when a thiosulfate or a sulfite is compounded, the thiosulfate or the sulfite is not uniformly dispersed and precipitates as a crystal, and thus an effect is less likely to be exhibited. The present inventors have found that coexistence of glycerin with such an inorganic salt enables uniform fine dispersion of a thiosulfate, a sulfite, or the like in a base to allow the reducing inorganic salt to exhibit an antioxidant effect, and thus the present invention has been completed.

Thiosulfates and sulfites are well known as reducing agents, and are substances that have been used for cosmetics, food, and medical applications from long ago and have many safety data. However, although it is assumed that adding the present substance to a transdermal preparation will impart a property of imparting stability to a medicine, the present substance has poor usability because it is an inorganic salt, and regarding use in a transdermal preparation, there has been one report (Japanese Patent No. 5856424) on a sulfite, but addition of a thiosulfate has not been known at all.

The present invention is as follows.
[1] A transdermal preparation including a base; a medicine; and a stabilizer, wherein the medicine and the stabilizer are contained in the base, and the stabilizer is selected from the group consisting of thiosulfates, sulfites, bisulfites, and nitrites.
[2] The transdermal preparation according to [1], wherein the base is one or more selected from the group consisting of acryl-based adhesives, rubber-based adhesives, and silicone-based adhesives.
[3] The transdermal preparation according to [1] or [2], having a content of the stabilizer of 0.01 to 2 wt% based on a total weight of a plaster containing the medicine.
[4] A transdermal preparation including a base; a medicine; and a stabilizer, wherein the medicine and the stabilizer are contained in the base, the stabilizer is selected from the group consisting of thiosulfates, sulfites, bisulfites, and nitrites, and the transdermal preparation further includes glycerin.
[5] The transdermal preparation according to [4], having a content of the glycerin of 0.01 to 20 wt% based on a total weight of a plaster containing the medicine.
[6] The transdermal preparation according to [4] or [5], wherein the medicine, the stabilizer, and the glycerin coexist in the base that is an adhesive, and an amount of the glycerin added is 1.5 to 2000 parts by weight based on 1 part by weight of the stabilizer.
[7] The transdermal preparation according to [4] or [5], further including an absorption enhancer, wherein the medicine, the stabilizer, and the glycerin coexist in the base that is an adhesive, and an amount of the glycerin added is 1.5 to 2000 parts by weight based on 1 part by weight of the stabilizer.
[8] The transdermal preparation according to any one of [1] to [7], wherein the medicine is rotigotine or a salt of rotigotine.
[9] The transdermal preparation according to any one of [1] to [3], further including an absorption enhancer, wherein the medicine is rotigotine or a salt of rotigotine.
[10] The transdermal preparation according to [9], wherein the absorption enhancer is selected from the group consisting of cetyl alcohol, lauric acid diethanolamide, isopropyl myristate (IPM), isopropyl palmitate (IPP), and stearic acid.
[11] The transdermal preparation according to [9] or [10], wherein the medicine is rotigotine or a salt of rotigotine, and the base that is an adhesive obtained by mixing polyisobutylene (PIB) and an acryl-based adhesive contains cetyl alcohol.
[12] The transdermal preparation according to [9] or [10], wherein the medicine is rotigotine or a salt of rotigotine, and the base that is an adhesive obtained by mixing a styrene-isoprene-styrene block copolymer (SIS) and an acryl-based adhesive contains IPM.
[13] The transdermal preparation according to any one of [1] to [7], wherein the medicine is fentanyl or a salt of fentanyl.
[14] The transdermal preparation according to any one of [1] to [3], further including an absorption enhancer, wherein the medicine is fentanyl or a salt of fentanyl.
[15] The transdermal preparation according to [14], wherein the absorption enhancer is selected from the group consisting of cetyl alcohol, lauric acid diethanolamide, isopropyl myristate (IPM), isopropyl palmitate (IPP), and stearic acid.
[16] The transdermal preparation according to [14] or [15], wherein the medicine is fentanyl or a salt of fentanyl, and the base is an adhesive including a styrene-isoprene-styrene block copolymer (SIS) .
[17] A method for producing a transparent or semitransparent coating solution, the method including a step of mixing a medicine, a stabilizer selected from the group consisting of thiosulfates, sulfites, bisulfites, and nitrites, and glycerin in a rubber-based and acryl-based adhesive solution.
[18] A method for producing a coating liquid, the method including the steps of: making a medicine, a stabilizer selected from the group consisting of thiosulfates, sulfites, bisulfites, and nitrites, and glycerin coexist in a rubber-based and acryl-based adhesive solution; and mixing a sucrose fatty acid ester.

### EFFECT OF THE INVENTION

Thiosulfates, sulfites, bisulfites, and nitrites (hereinafter, sometimes referred to as "thiosulfate or the like") as stabilizers included in the transdermal preparation of the present invention are neutral compounds having a reducing anion. In a case where the medicine is a basic compound, it is considered that the medicine interacts with the anion of a thiosulfate or the like and is stabilized to suppress degradation. Furthermore, using a combination of a thiosulfate or the like and a known absorption enhancer extremely improves the release amount of the medicine per unit area and unit time and the migration property of the medicine into the skin. This is considered to be because the absorption enhancer changes the physical property of the base and penetrates the skin to deteriorate the barrier function of the stratum corneum. As a result, the distribution coefficient of the medicine between the base and the skin changes, or the diffusion rate of the medicine in the skin increases, to improve the release amount of the medicine, and a required amount of the medicine easily penetrates the skin and is absorbed into the internal circulatory system.

Therefore, in the case of a tape preparation, a transdermal preparation can be obtained that has a larger effective dose than a conventional medicine-containing transdermal preparation having the same area. In other words, the same effect as that of a conventional product can be obtained with a transdermal preparation having a smaller area than the conventional product.

Thus, occurrence of erythema can be prevented in persons who are sensitive to skin irritation, or the area of erythema is reduced. Furthermore, the area of the preparation can be reduced, and therefore the adhering operation is easy and causes no discomfort.

### BRIEF DESCRIPTION OF THE DRAWINGS

FIG. 1 is a chromatogram of a rotigotine patch extract of Comparative Example 2.
FIG. 2 is a chromatogram of a rotigotine patch extract of Example 1.
FIG. 3A is a chromatogram of a fentanyl patch extract of Example 3, and FIG. 3B is a chromatogram of a fentanyl patch extract of Comparative Example 4.

### MODE FOR CARRYING OUT THE INVENTION

The transdermal preparation of the present invention is a transdermal preparation including a medicine and a stabilizer in a base. The stabilizer in the transdermal preparation of the present invention is selected from the group consisting of thiosulfates, sulfites, bisulfites, and nitrites. The stabilizer is one or more selected from the group consisting of thiosulfates, sulfites, bisulfites, and nitrites. Examples of the salt in each stabilizer include salts with an alkali metal and salts with an alkaline earth metal, and sodium salts are preferable. That is, the thiosulfate is preferably sodium thiosulfate, the sulfite is preferably sodium sulfite, the bisulfite is preferably sodium bisulfite, and the nitrite is preferably sodium nitrite. The stabilizer such as a thiosulfate may be used alone or in combination with a known absorption enhancer. The content of the stabilizer in the base (the total weight of the plaster containing the medicine) is preferably 0.01 to 5 wt%, and more preferably 0.02 to 2 wt%. If the content of the stabilizer in the base is less than 0.01 wt%, the stabilization effect on the medicine is weak, and if the content is more than 5 wt%, a problem tends to occur in crystal precipitation in the base.

Another feature of the present invention is addition of a salt dissolution aid in compounding the stabilizer such as a thiosulfate into the base. Inorganic salts such as Na thiosulfate and Na sulfite have extremely high polarity, and therefore have poor compatibility with rubber-based adhesives and acryl-based adhesives. Furthermore, such inorganic salts are completely insoluble in solvents of these adhesives, such as ethyl acetate, toluene, and cyclohexane, and therefore it is extremely difficult to dissolve the medicine and the adhesive and disperse the thiosulfate or the like uniformly in the solution. The present inventors have found that when a thiosulfate or the like is to be uniformly and finely dispersed in an adhesive solution, coexistence of glycerin with the thiosulfate or the like enables uniform fine dispersion in the adhesive solution, and even in a dried transdermal preparation, the thiosulfate or the like is stably and finely dispersed in the base to exhibit an effect of stabilizing the medicine. That is, the transdermal preparation of the present invention preferably further includes glycerin in the base. In the transdermal preparation of the present invention, the medicine, the stabilizer, and the glycerin preferably coexist in the base.

The content of the glycerin is 0.01 to 20 wt%, more preferably 0.02 to 10 wt%, and particularly preferably 0.1 to 5 wt% in the base (the total weight of the plaster containing the medicine). If the content of the glycerin in the base is less than 0.01 wt%, the effect of stabilizing the solution is weak at the time of uniformly dispersing the thiosulfate or the like in the base or the coating solution for production, and if the content is more than 20 wt%, bleeding occurs on the surface of the adhesive plaster, and an inappropriate matter such as a decrease in the adhesive strength tends to occur. Furthermore, the amount of the glycerin added is preferably 1.5 to 2000 parts by weight based on 1 part by weight of the stabilizer.

In the transdermal preparation of the present invention, an absorption enhancer can be used in combination. The transdermal preparation of the present invention preferably further includes an absorption enhancer in the base. In the transdermal preparation of the present invention, the medicine, the stabilizer, the glycerin, and the absorption enhancer preferably coexist in the base. Examples of the absorption enhancer include 1-dodecylcycloheptan-2-one, oleyl alcohol, cetyl alcohol, oleic acid, stearic acid, lauric acid, sodium lauryl sulfate, lauric acid methanolamide, lauric acid diethanolamide, 1-methyl-2-pyrrolidone, isopropyl myristate (IPM), isopropyl palmitate (IPP), triacetin, lactic acid, and polyoxyethylene alkyl ether phosphates. The absorption enhancer is preferably selected from the group consisting of cetyl alcohol, lauric acid diethanolamide, isopropyl myristate (IPM), isopropyl palmitate (IPP), and stearic acid. In order to promote absorption of a basic medicine, the absorption enhancer is preferably triacetin, diisopropanolamine, or the like. The content of the absorption enhancer in the base (the total weight of the plaster containing the medicine) is preferably 1 to 20 wt%. If the amount of the absorption enhancer added into the base is less than 1 wt%, the promoting effect is weak, and if the amount is more than 20 wt%, a problem tends to occur that the action on the skin is so strong that irritation occurs.

The amount of the added medicine or the added absorption enhancer contained in the base corresponds to the addition amount with respect to the preparation itself in the case of an ointment, a cream, or a liniment, corresponds to the addition amount with respect to the portion excluding the support and the release paper from the preparation, that is, the adhesive base portion in the case of a tape preparation or a cataplasm, and corresponds to the addition amount with respect to the base contained in the reservoir layer and/or the adhesive layer in the case of a patch preparation.

The base of the transdermal preparation used in the present invention is an adhesive base in a case where the transdermal preparation is a tape preparation or a cataplasm, and is a non-adhesive base in a case where the transdermal preparation is an ointment, a cream, a patch preparation, or a liniment.

The adhesive base is not particularly limited as long as it has a general adhesive composition such that the adhesive base can dissolve a medicine and has pressure-sensitive adhesion so as to fix to the skin or the mucosa at normal temperature for a long time. Examples of the preferable adhesive base include bases including an acryl-based adhesive, bases including a rubber-based adhesive, and bases including a silicone-based adhesive. That is, in a case where the base is the adhesive base, the base is preferably selected from the group consisting of acryl-based adhesives, rubber-based adhesives, and silicone-based adhesives. These adhesives may be used singly or in combination of two or more kinds thereof.

Examples of the acryl-based adhesive used in the acryl-based adhesive base include homopolymers and copolymers of alkyl (meth)acrylates obtained from an aliphatic alcohol having 1 to 18 carbon atoms and particularly preferably 4 to 18 carbon atoms and (meth)acrylic acid, and copolymers of an alkyl (meth)acrylate and another functional monomer.

Examples of the functional monomer include monomers having a hydroxyl group, monomers having a carboxyl group, monomers having an amide group, and monomers having an amino group, and copolymerizable monomers can also be used such as vinyl acetate, styrene, α-methylstyrene, vinyl chloride, acrylonitrile, ethylene, propylene, and butadiene. The adhesive preferably contains 50 wt% or more of alkyl (meth)acrylate as a (co)polymerization component.

In order to prepare an acryl-based adhesive, solution polymerization of a required monomer is usually performed in the presence of a polymerization initiator. However, the polymerization form is not limited thereto. The polymerization reaction conditions are appropriately selected mainly according to the kind of the monomer.

Examples of the rubber-based adhesive used in the rubber-based adhesive base include adhesives obtained by adding 20 to 200 parts by weight of a tackifier such as a rosin-based resin, a polyterpene resin, a coumarone-indene resin, a petroleum-based resin, or a terpene-phenol resin and, if necessary, an appropriate amount of agent such as a softener such as liquid polybutene, mineral oil, lanolin, liquid polyisoprene, or liquid polyacrylate, a filler such as titanium oxide, or an anti-aging agent such as butylhydroxytoluene to 100 parts by weight of a rubber elastic body such as natural rubber, a styrene-isoprene-styrene block copolymer (SIS), a styrene-butadiene-styrene block copolymer, a styrene-olefin-styrene block copolymer, polyisoprene, polybutene, polyisobutylene (PIB), or an ethylene-vinyl acetate copolymer.

Examples of the silicone-based adhesive used in the silicone-based adhesive base include adhesives containing polydimethylsiloxane or the like as a main component.

A compounding agent such as a plasticizer, a filler, or an anti-aging agent may be added to the adhesive (to the adhesive base) as necessary.

Examples of the non-adhesive base include beeswax, fat and oil, lanolin, white petrolatum, paraffin, Plastibase, higher fatty acids, higher alcohols, emulsifiers, Macrogol, and carboxyvinyl polymers.

The medicine (bioactive substance) used in the present invention is to be capable of penetrating a biological membrane transdermally.

Examples of the medicine include antipyretics, analgesics and anti-inflammatory agents, steroidal anti-inflammatory agents, non-steroidal anti-inflammatory agents, narcotic analgesics, antiepileptic agents, antipsychotic agents, antidepressant/anxiolytic agents, antiparkinsonian agents, anti-dementia agents, hypnotic agents, sedative agents, antispasmodic agents, muscle relaxants, autonomic agents, cerebral circulation/metabolism improving agents, cardiotonic agents, antianginal agents, antihypertensive/antiarrhythmic agents, vasodilators, antihypertensive agents, vasopressors, diuretic agents, respiratory stimulants, antitussive and expectorant agents, bronchodilators, therapeutic agents for asthma/allergic rhinitis, respiratory agents, agents for common cold, antiemetics, antacids, antiulcer agents, laxatives, antidiarrheal agents, liver agents, therapeutic agents for pancreatic diseases, cholagogues, female hormones, male hormones, hypothalamic and pituitary hormones, ovulation inducing agents, therapeutic agents for diabetes insipidus, thyroid hormones, protein anabolic hormones, anti-thyroid agents, calcium metabolic agents, anti-inflammatory enzyme agents, antihistamine agents, antirheumatic agents, therapeutic agents for gout, hypoglycemic agents, vitamins, hematopoietic agents, hemostatic agents, therapeutic agents for hyperlipidemia, antibiotics, antitumor agents, immunosuppressive agents, antidotes, emetics agent, anthelmintics, antiprotozoal agents, therapeutic agents for hemorrhoid, genitourinary agents, local anesthetics, and anticoagulants.

Examples of the antipyretics, analgesics and anti-inflammatory agents include indomethacin, salicylic acid, glycol salicylate, aspirin, acetaminophen, diclofenac sodium, ibuprofen, sulindac, naproxen, and ketoprofen.

Examples of the steroidal anti-inflammatory agents include hydrocortisone, prednisolone, fluocinolone acetonide, fludroxycortide, and methylprednisolone.

Examples of the narcotic analgesics include morphine, oxycodone, and fentanyl. Fentanyl may be free or in a form of a salt, and may be an inorganic salt or an organic salt as long as it is a pharmaceutically acceptable salt. Examples of the salt include fentanyl citrate. In the present invention, fentanyl citrate is preferably used.

Examples of the vasodilators include diltiazem, verapamil, pentaerythritol tetranitrate, dipyridamole, isosorbide dinitrate, nifedipine, and nitroglycerin.

Examples of the antihypertensive/antiarrhythmic agents include propanolol, atenolol, pindolol, quinidine sulfate, ajmaline, alprenolol hydrochloride, metoprolol tartrate, nadolol, timolol maleate, and disopyramide.

Examples of the antihypertensive agents include clonidine hydrochloride, captopril, and bunitrolol hydrochloride.

Examples of the antitussive and expectorant agents include procaterol hydrochloride and terbutaline sulfate.

Examples of the antitumor agents include 5-fluorouracil, 1-(2-tetrahydrofuryl)-5-fluorouracil, and mitomycin C.

Examples of the local anesthetics include benzocaine, procaine, lidocaine, and tetracaine.

Examples of the hormones include steroid hormones such as estrogen, estradiol, testosterone, progesterone, and prostaglandins and peptide hormones such as insulin.

Examples of the therapeutic agents for asthma/allergic rhinitis include ketotifen fumarate, azelastine hydrochloride, and sodium cromoglycate.

Examples of the antihistamine agents include cycloheptadine hydrochloride, diphenhydramine hydrochloride, phenbenzamine, and mequitazine.

Examples of the anticoagulants include heparin.

Examples of the antispasmodic agents include scopolamine and clofluperol.

Examples of the cerebral circulation/metabolism improving agents include vinpocetine, flunarizine hydrochloride, nicardipine hydrochloride, brovincamine fumarate, dihydroergotoxine mesylate, ifenprodil tartrate, and isoxsuprine hydrochloride.

Examples of the antidepressant/anxiolytic agents include maprotiline hydrochloride, etizolam, diazepam, bromazepam, amitriptyline hydrochloride, and mianserin hydrochloride.

Examples of the anti-dementia agents include donepezil and rivastigmine.

Examples of the antiparkinsonian agents include ropinirole (hydrochloride) and rotigotine. Rotigotine may be free or in a form of a salt, and may be an inorganic salt or an organic salt as long as it is a pharmaceutically acceptable salt. Examples of the salt include rotigotine hydrochloride. In the present invention, free rotigotine is preferably used.

Examples of the vitamin D preparation include alfacalcidol and ergocalciferol.

Examples of the hypoglycemic agents include glibenclamide and gliclazide.

Examples of the antiulcer agents include clebopride malate, famotidine, and glycopyrronium bromide.

Examples of the hypnotic agents include phenobarbital and amobarbital.

Examples of the antibiotics include tetracycline and chloramphenicol.

In the present invention, the transdermal absorption of various medicines can be promoted. Therefore, by combining a medicine with the composition of the present invention, a preparation having excellent transdermal absorption can be produced whether the medicine is a basic medicine or an acidic medicine, but a basic medicine is desirably selected from the viewpoint of using a thiosulfate or the like as a stabilizer. The basic medicine is a medicine that has a basic functional group such as an amino group (primary, secondary, or tertiary) in the molecule and exhibits basicity as a compound, and the acidic medicine is a medicine that has an acidic functional group such as a carboxy group in the molecule and exhibits acidity as a compound. Rotigotine for Parkinson disease and salts of rotigotine, and fentanyl as an analgesic and salts of fentanyl are also examples of the basic compound. The medicine is also preferably rotigotine or a salt of rotigotine, and is also preferably fentanyl or a salt of fentanyl.

The amount of such a medicine added depends on the kind of the medicine, the purpose of use of the transdermal preparation, and the like, but is preferably 0.1 to 30 wt% in the base (the total weight of the plaster containing the medicine). The saturated solubility of the medicine in the base depends on the composition of the base. By making the medicine compatible in the base at a concentration as close as possible to the saturated solubility without crystal precipitation, high releasability of the medicine is obtained. However, precipitation of a crystal of the medicine in the base causes no problem. It is also possible to encapsulate the medicine or the absorption enhancer or to provide a storage layer of the medicine or the absorption enhancer.

The transdermal preparation used in the present invention is a tape preparation, a cataplasm, a patch preparation, a cream, a liniment, an ointment, or the like.

However, the medicine, the stabilizer, and the like in the reservoir layer may also be contained in the adhesive layer.

The cream, the ointment, and the liniment are a medicine-containing paste, slurry, or liquid obtained by uniformly mixing the medicine, the stabilizer, and an additive such as an absorption enhancer added as necessary with the non-adhesive base.

The tape preparation is obtained by providing the adhesive base containing the medicine, the stabilizer, and an additive such an absorption enhancer added as necessary on one surface of a support, and preferable adhesive bases have good adhesion to the skin.

The cataplasm is obtained by applying, in a layer form, the adhesive base containing the medicine, the stabilizer, water, and an additive such an absorption enhancer added as necessary on one surface of a support, and preferable adhesive bases have good affinity with water. In a case where the adhesive base has poor adhesiveness, the adhesive base is fixed to the skin surface with an adhesive plaster, an adhesive tape, or the like.

The patch preparation is formed by sequentially layering a non-adhesive reservoir layer and an adhesive layer including the adhesive on one surface of a support, and the non-adhesive base containing the medicine, the stabilizer, and an additive such as an absorption enhancer added as necessary is retained in the reservoir layer. This reservoir layer is adhered to the skin via the adhesive layer, and the medicine in the reservoir layer is transdermally absorbed through the adhesive layer.

A fat-soluble solubilizer, purified water, a water-soluble solubilizer, a pH adjuster, and the like may be added to the cream and the ointment. Examples of the fat-soluble solubilizer include liquid paraffin, isopropyl myristate, and diethyl sebacate, and examples of the water-soluble solubilizer include ethanol, glycerin, and propylene glycol.

As the supports of the tape preparation, the cataplasm, and the patch preparation, supports are used that are flexible but impart a self-supporting property to the transdermal preparation and serve to prevent the medicine in the adhesive base layer or the reservoir layer from volatilizing and migrating. Examples of the material of the support include cellulose acetate, ethyl cellulose, polyethylene terephthalate, a plasticized vinyl acetate-vinyl chloride copolymer, nylon, an ethylene-vinyl acetate copolymer, plasticized polyvinyl chloride, polyurethane, polyethylene, polyvinylidene chloride, and aluminum. These materials are used in a form of, for example, a single layer sheet or film, or a laminate of two or more sheets. The materials other than aluminum may be used in a form of a woven fabric or a nonwoven fabric. As the support, a support is preferably used that is made of a material having followability to the skin surface, and a laminate film of polyethylene terephthalate and an ethylene-vinyl acetate copolymer is particularly preferable. The support preferably has a thickness of 5 to 100 um.

A tape preparation is formed by forming an adhesive base layer on one surface of the support, and a patch preparation is formed by sequentially layering a reservoir layer and an adhesive layer on one surface of the support. In the patch preparation, a suitable control film may be present between the reservoir layer and the adhesive layer.

When the tape preparation is prepared, an ordinary method of producing an adhesive tape can be applied to form the adhesive base layer. A typical example of the method is a solvent coating method, and in addition, a hot melt coating method, an electron beam curing emulsion coating method, and the like are used. In order to form the adhesive base layer with a solvent coating method, for example, the medicine, the stabilizer, and an additive such as an absorption enhancer added as necessary are dissolved or dispersed in an appropriate solvent, and the obtained solution or dispersion is directly applied to one surface of a support and dried to form an adhesive base layer having a required thickness. Alternatively, the solution or dispersion may be applied to release paper for protection, and the adhesive base layer obtained after drying may be brought into close contact with a support. The thickness of the adhesive base layer depends on the purpose of use, but is preferably 10 to 200 um.

The tape preparation is usually provided with release paper on its adhering surface in order to protect the surface of the adhesive base layer until the time of use. The patch preparation is provided with release paper on the adhering surface of the adhesive layer. As the release paper, release paper is often used that is obtained by subjecting a polyethylene terephthalate film to silicone treatment, but the release paper is not limited thereto. The release paper has a thickness of 1000 um or less, and preferably 30 to 200 um.

In order to produce a cataplasm, the adhesive base, the medicine, the stabilizer, water, and an additive such as an absorption enhancer added as necessary are uniformly mixed, and the obtained medicine-containing paste is applied in a layer form on one surface of a support. To the medicine-containing paste, another additive such as purified water, a humectant, an inorganic filler, a viscosity modifier, a crosslinking agent, or an anti-aging agent may be further added. Examples of the humectant include glycerin and propylene glycol, and examples of the inorganic filler include kaolin, bentonite, zinc oxide, and titanium dioxide.

The obtained transdermal preparations in various dosage forms are usually adhered or applied to the surface of the skin or the mucosa directly for the purpose of transdermally or transmucosally administering the medicine to the internal circulatory system. Furthermore, these transdermal preparations are sometimes adhered or applied to the skin or the mucosa for the purpose of treating a diseased part of the skin or the mucosa with the medicine.

### EXAMPLES

Hereinafter, the present invention will be described more specifically with reference to Examples. Note that the present invention is not limited to these Examples, and various modifications can be made without departing from the technical concept of the present invention.

Hereinafter, the term "part(s)" means "part(s) by weight". The evaluation methods and the measurement methods for the skin migration test, the skin irritation test, and the skin penetration test shown in the results are as follows.

### Preparation of transdermal preparation

### (Rotigotine transdermal preparation)

The method for producing a transdermal preparation in Example 1 is as follows. In Examples 0 and 2 and Comparative Examples, a transdermal preparation was produced in accordance with the method in Example 1. Table 1 shows the compositions.

An aqueous solution of a mixture of predetermined amounts of cyclohexane solutions of (1) and (2), an ethyl acetate solution of (3), toluene solutions of (5) and (6), (7), (8), and (9) was prepared. To the aqueous solution, predetermined amounts of the ethyl acetate solution of (3), an ethanol solution of (11), and a toluene solution of (12) were added. A THF solution of (4) was further added, and the resulting solution was stirred well and mixed to obtain a coating solution. The coating solution was applied to a PET film having a thickness of 40 um and dried to obtain a tape transdermal preparation in which an adhesive layer having a thickness of 50 um was layered.

**[Table 1]**

| Component | Mixing ratio (parts) | | | | | |
|---|---|---|---|---|---|---|
| | Ex. 0 | Ex. 1 | Comp.Ex.1 | Ex. 2 | Comp.Ex.2 | |
| High molecular weight PIB (Oppanol B50SF, BASF) | 42 | 42 | 42 | 37 | 37 | (1) |
| Low molecular weight PIB (Oppanol B12SFN, BASF) | 2 | 2 | 2 | 2 | 2 | (2) |
| Acryl adhesive (MAS811B, CosMED Pharmaceutical Co. Ltd.) | 10 | 10 | 10 | 15 | 15 | (3) |
| Rotigotine (Rotigotine (S-ROT-3), Shandong Keyuan Pharmaceutical Co., Ltd.) | 10 | 10 | 10 | 10 | 10 | (4) |
| Liquid paraffin (SMOIL P-350P, MORESCO Corporation) | 15 | 1 5 | 15 | 15 | 15 | (5) |
| Alicyclic saturated hydrocarbon resin (ARKON P-100, ARAKAWA CHEMICAL INDUSTRIES, LTD.) | 15 | 15 | 15 | 15 | 15 | (6) |
| BHT (Dibutylhydroxytoluene, MercK KGaA) | 1 | 1 | 1 | 1 | 1 | (7) |
| Na thiosulfate | 0. 2 | 0. 2 | 0. 005 | 0. 3 | - | (8) |
| Glycerin | - | 0. 8 | - | 1. 0 | - | (9) |
| Ascorbyl palmitate | 0.1 | 0.1 | 0. 1 | 0. 1 | 0.1 | (10) |
| Sucrose fatty acid ester | 2 | 2 | 2 | 2 | 2 | (11) |
| Cetyl alcohol | 2. 8 | 2 . 8 | 2. 8 | 2. 8 | 2. 8 | (12) |

**[Table 2]**

| Observation of dispersion state of coating solution | | | | | |
|---|---|---|---|---|---|
| Time of leaving coatinq solution | Ex.0 | Ex. 1 | Comp.Ex. 1 | Ex. 2 | Comp.Ex. 2 |
| 0 hours | Milk white dispersion | Transparent solution | Milk white dispersion | Transparent solution | Milk white dispersion |
| 24 hours | Crystal is present | Transparent solution | Crystal is present | Transparent solution | Crystal is present |

### (Fentanyl transdermal preparation)

The method for producing a transdermal preparation in Example 3 is as follows. In Example 4 and Comparative Examples, a transdermal preparation was produced in accordance with the method in Example 3. Table 3 shows the compositions.

Predetermined amounts of a SIS polymer, an alicyclic saturated hydrocarbon resin, and liquid paraffin were dissolved in cyclohexane to obtain a base solution. A predetermined amount of lauric acid diethanolamide was dissolved in methanol, and a predetermined amount of fentanyl citrate was further added to obtain a medicine solution. The medicine solution was added to the base solution, an aqueous sodium sulfite solution or an aqueous sodium bisulfite solution was added thereto, glycerin was further added, and the resulting solution was stirred well and mixed to obtain a coating solution. The coating solution was applied to a PET film having a thickness of 40 um and dried to obtain a tape transdermal preparation in which an adhesive layer having a thickness of 50 um was layered.

**[Table 3]**

| Component | Mixing ratio (parts) | | |
|---|---|---|---|
| | Ex. 3 | Ex. 4 | Comp.Ex.3 |
| SIS polymer (Kraton D1161 JSP, Kraton JSR Elastomers K.K.) | 22.5 | 22.5 | 22.5 |
| Alicyclic saturated hydrocarbon resin (ARKON P-100, ARAKAWA CHEMICAL INDUSTRIES, LTD.) | 42.5 | 42.5 | 42.5 |
| Liquid paraffin (SMOIL P-350P, MORESCO Corporation) | 20.0 | 20.0 | 20.0 |
| Fentanyl citrate (Kern) | 5.0 | 5.0 | 5.0 |
| Lauric acid diethanolamide (PROFAN AA-62EX, Sanyo Chemical Industries, Ltd.) | 1.0 | 1.0 | 1.0 |
| Glycerin (First grade, Wako Pure Chemical Industries, Ltd.) | 1.0 | 1.0 | - |
| Sodium sulfite (Wako Pure Chemical Industries, Ltd.) | 0.2 | - | 0.2 |
| Sodium bisulfite (Wako Pure Chemical Industries, Ltd.) | - | 0.05 | |

**[Table 4]**

| Observation of dispersion state of coating solution with microscope | | | |
|---|---|---|---|
| Time of leaving coating solution | Ex. 3 | Ex. 4 | Comp.Ex. 3 |
| Immediately after | Crystal is absent | Crystal is absent | Crystal is absent |
| 24 hours | Crystal is absent | Crystal is absent | Crystal is present |

### (Rotigotine transdermal preparation)

A transdermal preparation containing rotigotine in a mixed adhesive base of a styrene-isoprene-styrene block copolymer (SIS) and an acryl-based adhesive or in an acryl-based adhesive was prepared in accordance with the method in Example 1. Table 5 shows the compositions.

**[Table 5]**

| Component | Mixing ratio (parts) | | | |
|---|---|---|---|---|
| | Ex. 5 | Comp.Ex.4 | Ex. 6 | Comp.Ex.5 |
| SIS polymer (Kraton D1161 JSP) | 47. 5 | 47. 5 | - | - |
| Acryl adhesive (MAS811B, CosMED Pharmaceutical Co. Ltd.) | 8 | 8 | 8 2 | 8 2 |
| Rotigotine | 9 | 9 | 12 | 12 |
| Liquid paraffin | 14 | 14 | - | - |
| Alicyclic saturated hydrocarbon resin | 1 4 | 14 | - | - |
| Na thiosulfate | 0. 2 | - | 0. 5 | - |
| Glycerin | 0. 67 | 0. 67 | 2. 0 | 2. 0 |
| Ascorbyl palmitate | 0. 02 | 0. 02 | 0. 0 2 | 0. 02 |
| IPM | 3 | 3 | - | - |
| IPP | 3 | 3 | - | - |
| Lauric acid diethanolamide | - | - | 3 | 3 |

The medicine transdermal absorption test and the medicine stability test were performed as follows.

### Medicine transdermal absorption test

A human excised skin was sandwiched in a Franz diffusion cell in which water at 37°C was circulated, and a PBS buffer solution (pH: 7.4) was supplied to the receiver (dermis) side and stirred with a magnetic stirrer. The obtained transdermal preparation was applied to the donor (keratin) side, and a penetration test was performed. After 12 hours and 24 hours, the mixed liquid in the receiver was collected, the concentration of the medicine in the mixed liquid was measured with a high performance liquid chromatograph (HPLC), and the cumulative amount of the medicine that had penetrated the skin was determined. The cumulative amount of the medicine was the average of the test results with n = 3.

### Medicine stability test

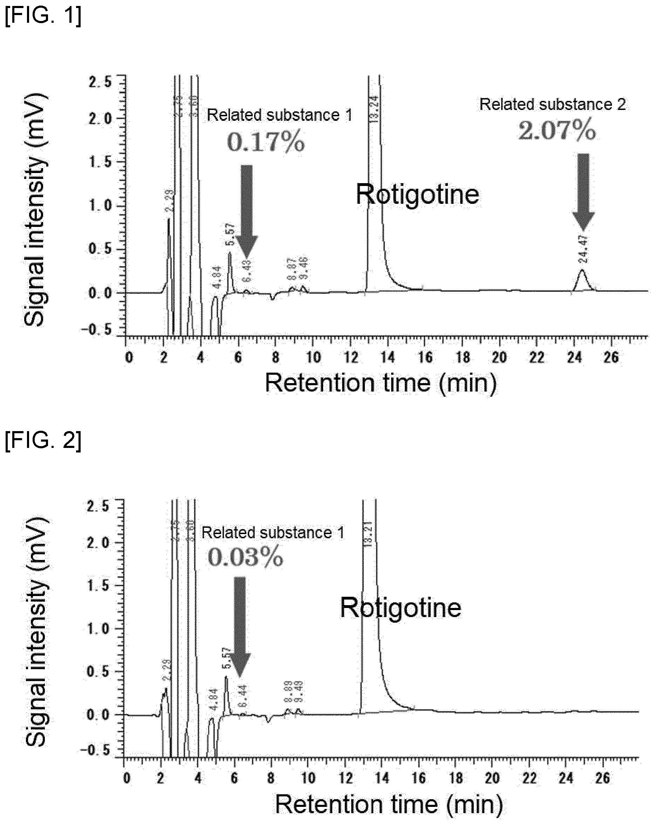

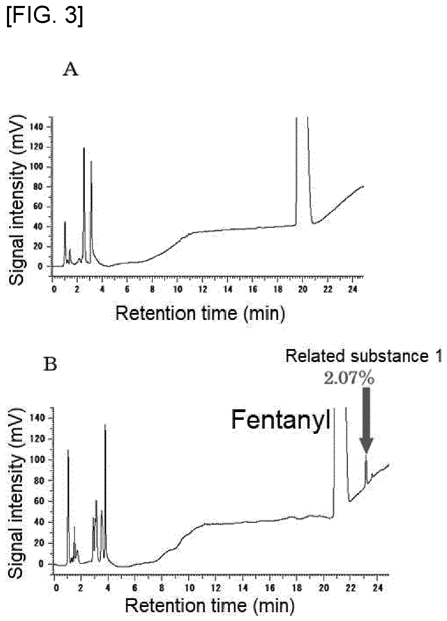

The prepared tape preparation was punched into a circle having a diameter of 2 cm, enclosed in an aluminum laminate bag, and stored at 60°C for 1 week in Examples 1 and 2 and Comparative Examples 1 and 2. The tape preparation was stored for 3 days in Examples 3 and 4 and Comparative Examples 1 and 2. The tape preparation was stored at 60°C for 4 weeks in Examples 5 and 6 and Comparative Examples 3 and 4. Thereafter, the tape preparation was immersed in ethanol or the like for 4 hours to extract the medicine, and the medicine content was measured with an HPLC. Due to the heat storage, a peak of a related substance derived from a substance considered to be a related substance of the medicine appeared. As a measure of stability, the expression level of the related substance peak was measured simultaneously with the medicine content. FIG. 1 shows the state of expression of related substances of rotigotine as a chromatogram from the test result on the sample of Comparative Example 2, and FIG. 2 shows a chromatogram of Example 1. Similarly, FIG. 3B shows the state of expression of a related substance of fentanyl as a chromatogram from the test result on the sample of Comparative Example 4, and FIG. 3A shows a chromatogram of Example 3. In FIGS. 1 to 3, the horizontal axis represents the retention time (min), and the vertical axis represents the signal intensity (mV).

### Rotigotine HPLC conditions

Detector: Ultraviolet absorption photometer (measurement wavelength: 225 nm)
Column: Stainless steel tube having inner diameter of 4.6 mm and length of 25 cm, filled with octadecylsilyl silica gel for liquid chromatography of 5 um (CAPCELL PAK C18 or column having performance equivalent thereto)
Pre-column: SecurityGuard C8, 3.0 mm × 4.0 mm, 5 um (Phenomenex) Column temperature: Constant temperature around 40°C
Mobile phase A: Buffer solution (pH: 4.5, 100 mL) of 50 mM NaH₂PO₄ and 2.5 mM Na 1-octanesulfonate, into which 53.8 mL of acetonitrile was compounded.
Flow rate: 1.0 mL per minute
Sample temperature: Constant temperature around 4°C
Area measurement range: Up to 25 minutes after injection

### Fentanyl HPLC conditions

Detector: Ultraviolet absorption photometer (measurement wavelength: 210 nm)
Column: Stainless steel tube having inner diameter of 3.0 mm and length of 10 cm, filled with octylsilyl silica gel for liquid chromatography of 3 um (InertSustain C8 HP or column having performance equivalent thereto)
Pre-column: SecurityGuard C8, 3.0 mm × 4.0 mm, 5 um (Phenomenex) Column temperature: Constant temperature around 25°C
Mobile phase A: Phosphate buffer (0.02 mol/L, pH: 2.5)
Mobile phase B: Acetonitrile
Feeding of mobile phase: Concentration gradient control by changing mixing ratio of mobile phases A and B as follows

**[Table 6]**

| Time after injection (min) | | Mobile phase A (vol%) | Mobile phase B (vol%) | |
|---|---|---|---|---|
| 0.00 | | 97.0 | 3.0 | |
| 4.00 | | 95.0 | 5.0 | |
| 8.00 | | 80.0 | 20.0 | |
| 18.00 | | 75.0 | 25.0 | |
| 30.00 | | 20.0 | 80.0 | |
| 36.00 | | 20.0 | 80.0 | |
| 36.10 | | 97.0 | 3.0 | |
| 40.00 | | 97.0 | 3.0 | |

Flow rate: 0.6 mL per minute
Sample temperature: Constant temperature around 4°C
Area measurement range: Up to 36 minutes after injection

Using the obtained tape transdermal preparation, the medicine transdermal absorption test and the medicine stability test were performed. Table 7 shows the results.

**[Table 7]**

| Example, Comparative Example | Medicine transdermal absorption test (penetration amount after 24 hours(µg/cm²)) | | | Stability test (related substance (% with respect to main agent) | |
|---|---|---|---|---|---|
| | | Generation of crystal in coating solution | Generation of crystal in transdermal preparation | | |
| | | | | 6 min peak | 23 min peak |
| Ex. 0 | | ○ | ○ | 0.01 | 0 |
| Ex. 1 | 212.3 | ○ | ○ | 0.03 | 0 |
| Comp.Ex.1 | 198.2 | × | × | 0.07 | 0.75 |
| Ex. 2 | 220.1 | ○ | ○ | 0.02 | 0 |
| Comp.Ex.2 | | ○ | ○ | 0.17 | 2.07 |
| Ex. 5 | - | ○ | ○ | 0.11 | 0 |
| Comp.Ex.3 | - | ○ | ○ | 0.17 | 2.3 |
| Ex. 6 | - | ○ | ○ | 0.12 | 0.02 |
| Comp.Ex.4 | - | ○ | ○ | 0.18 | 3.0 |

| | | | | | |
|---|---|---|---|---|---|
| × Crystal precipitation is recognized. ∘ Slight turbidity is recognized, but precipitation and dispersion of crystal are not recognized. | | | | | |

From Table 7, it is found that addition of a stabilizer such as thiosulfate and glycerin significantly improves the medicine stability of the rotigotine transdermal preparation.

## Claims

1. A transdermal preparation comprising:
a base;
a medicine; and
a stabilizer,
wherein
the medicine and the stabilizer are contained in the base, and
the stabilizer is selected from the group consisting of thiosulfates, sulfites, bisulfites, and nitrites.

2. The transdermal preparation according to claim 1, wherein the base is one or more selected from the group consisting of acryl-based adhesives, rubber-based adhesives, and silicone-based adhesives.

3. The transdermal preparation according to claim 1 or 2, having a content of the stabilizer of 0.01 to 2 wt% based on a total weight of a plaster containing the medicine.

4. A transdermal preparation comprising:
a base;
a medicine; and
a stabilizer,
wherein
the medicine and the stabilizer are contained in the base,
the stabilizer is selected from the group consisting of thiosulfates, sulfites, bisulfites, and nitrites, and
the transdermal preparation further comprises glycerin.

5. The transdermal preparation according to claim 4, having a content of the glycerin of 0.01 to 20 wt% based on a total weight of a plaster containing the medicine.

6. The transdermal preparation according to claim 4 or 5, wherein
the medicine, the stabilizer, and the glycerin coexist in the base that is an adhesive, and
an amount of the glycerin added is 1.5 to 2000 parts by weight based on 1 part by weight of the stabilizer.

7. The transdermal preparation according to claim 4 or 5, further comprising an absorption enhancer, wherein
the medicine, the stabilizer, and the glycerin coexist in the base that is an adhesive, and
an amount of the glycerin added is 1.5 to 2000 parts by weight based on 1 part by weight of the stabilizer.

8. The transdermal preparation according to any one of claims 1 to 7, wherein the medicine is rotigotine or a salt of rotigotine.

9. The transdermal preparation according to any one of claims 1 to 3, further comprising an absorption enhancer, wherein
the medicine is rotigotine or a salt of rotigotine.

10. The transdermal preparation according to claim 9, wherein the absorption enhancer is selected from the group consisting of cetyl alcohol, lauric acid diethanolamide, isopropyl myristate (IPM), isopropyl palmitate (IPP), and stearic acid.

11. The transdermal preparation according to claim 9 or 10, wherein
the medicine is rotigotine or a salt of rotigotine, and
the base that is an adhesive obtained by mixing polyisobutylene (PIB) and an acryl-based adhesive contains cetyl alcohol.

12. The transdermal preparation according to claim 9 or 10, wherein
the medicine is rotigotine or a salt of rotigotine, and
the base that is an adhesive obtained by mixing a styrene-isoprene-styrene block copolymer (SIS) and an acryl-based adhesive contains IPM.

13. The transdermal preparation according to any one of claims 1 to 7, wherein the medicine is fentanyl or a salt of fentanyl.

14. The transdermal preparation according to any one of claims 1 to 3, further comprising an absorption enhancer, wherein
the medicine is fentanyl or a salt of fentanyl.

15. The transdermal preparation according to claim 14, wherein the absorption enhancer is selected from the group consisting of cetyl alcohol, lauric acid diethanolamide, isopropyl myristate (IPM), isopropyl palmitate (IPP), and stearic acid.

16. The transdermal preparation according to claim 14 or 15, wherein
the medicine is fentanyl or a salt of fentanyl, and
the base is an adhesive including a styrene-isoprene-styrene block copolymer (SIS).

17. A method for producing a transparent or semitransparent coating solution, the method comprising the step of mixing a medicine, a stabilizer selected from the group consisting of thiosulfates, sulfites, bisulfites, and nitrites, and glycerin in a rubber-based and acryl-based adhesive solution.

18. A method for producing a coating liquid, the method comprising the steps of:
making a medicine, a stabilizer selected from the group consisting of thiosulfates, sulfites, bisulfites, and nitrites, and glycerin coexist in a rubber-based and acryl-based adhesive solution; and
mixing a sucrose fatty acid ester.
